# EUROPÄISCHE PATENTSCHRIFT

(11) **EP 3 535 762 B1**
(45) Veröffentlichungstag und Bekanntmachung des Hinweises auf die Patenterteilung: **23.10.2024**
(21) Anmeldenummer: 17800701.9
(22) Anmeldetag: 03.11.2017
(51) Int. Cl.: G16H 40/63, G16H 40/40, A61M 1/14, A61M 1/36

(54) **EXTRAKORPORALES BLUTBEHANDLUNGSGERÄT SOWIE VERFAHREN ZUM AUSGEBEN EINER MELDUNG AN EINEM EXTRAKORPORALEN BLUTBEHANDLUNGSGERÄT**
EXTRACORPOREAL BLOOD TREATMENT DEVICE AND METHOD OF ISSUING A MESSAGE ON AN EXTRACORPOREAL BLOOD TREATMENT DEVICE
DISPOSITIF EXTRACORPOREL DE TRAITEMENT DU SANG ET MÉTHODE POUR ÉMETTRE UN MESSAGE SUR UN DISPOSITIF EXTRACORPOREL DE TRAITEMENT DU SANG

(30) Priorität: 03.11.2016 DE 102016013127
(43) Veröffentlichungstag der Anmeldung: 11.09.2019
(73) Patentinhaber: Fresenius Medical Care Deutschland GmbH, 61352 Bad Homburg (DE)
(72) Erfinder: BARDORZ, Christoph, 97228 Rottendorf (DE); NICHOLAS, Olaf, 97318 Kitzingen (DE); MÜLLER, Carsten, 97502 Euerbach (DE); TRAUNER, Karin Helga, 97422 Schweinfurt (DE)
(74) Vertreter: Herrmann, Uwe
(86) Internationale Anmeldenummer: PCT/EP2017/001279
(87) Internationale Veröffentlichungsnummer: WO 2018/082813

(56) Entgegenhaltungen:
- EP-A1- 1 838 356
- US-A1- 2016 151 592
- ANONYMOUS: "Dialog+ Dialysis Machine - Alarms and remedial action", 31 May 2016 (2016-05-31), XP055774809, Retrieved from the Internet <URL:https://www.bbraunusa.com/content/dam/b-braun/us/website/products-and-therapies/renal-therapies/clinical-support/dialog-v9-1x-ifu.pdf> [retrieved on 20210210]
- ANONYMOUS: "Dialog+ Dialysis Machine", 31 May 2016 (2016-05-31), XP055643203, Retrieved from the Internet <URL:https://www.bbraunusa.com/content/dam/b-braun/us/website/products-and-therapies/renal-therapies/clinical-support/dialog-v9-1x-ifu.pdf.bb-.50982881/dialog-v9-1x-ifu.pdf> [retrieved on 20191115]
- ROY THOMAS: "Patients' safety and haemodialysis devices", NEPHROLOGY DIALYSIS TRANSPLANTATION, vol. 16, no. 11, 1 November 2001 (2001-11-01), GB, pages 2138 - 2142, XP093114129, ISSN: 0931-0509, Retrieved from the Internet <URL:https://watermark.silverchair.com/162138.pdf?token=AQECAHi208BE49Ooan9kkhW_Ercy7Dm3ZL_9Cf3qfKAc485ysgAAA2gwggNkBgkqhkiG9w0BBwagggNVMIIDUQIBADCCA0oGCSqGSIb3DQEHATAeBglghkgBZQMEAS4wEQQMb-7yy0PT5CCFX3PBAgEQgIIDG_dYxWEQUyjaqLIr4Q_JGbI8UiG8LFlOV1IgbxBnAQrBcUxtaNULKM-C4gLDgjORTCbYfERLfN8fnuzz3SMy-qi7PsWTU> DOI: 10.1093/ndt/16.11.2138

## Beschreibung

Die vorliegende Erfindung betrifft ein extrakorporales Blutbehandlungsgerät mit wenigstens einer Ausgabeeinheit zur Ausgabe von zumindest einer Meldung an einen Nutzer des Blutbehandlungsgerätes.

Unter einem extrakorporalen Blutbehandlungsgerät wird im Rahmen der vorliegenden Erfindung ein Gerät zur Blutbehandlung verstanden, das über wenigstens einen extrakorporalen Blutkreislauf verfügt. In dem extrakorporalen Kreislauf erfolgt eine Behandlung des Blutes eines Patienten, wie z.B. die Blutreinigung oder die Auftrennung des Blutes in bestimmte Bestandteile.

Erfindungsgemäß handelt es sich bei dem Blutbehandlungsgerät um ein Dialysegerät.

Der Nutzer des Gerätes ist im Rahmen der vorliegenden Erfindung der Patient oder eine Person des Bedienungspersonals oder eine sonstige Person, die sich nicht in unmittelbarer Nähe des Gerätes aufhält, sondern z.B. in einer entfernt von dem Gerät angeordneten Überwachungszentrale.

Bei Dialysegeräten besteht die Notwendigkeit, periodisch einen sogenannten T1-Test durchzuführen, der die Funktionsüberprüfung der Schutzsysteme des Gerätes umfasst. Dieser Test wird jeweils beim Neustart des Gerätes durchgeführt, ausgelöst entweder durch den Nutzer des Gerätes oder durch das Gerät selbst. Der Test wird vor jeder Behandlung durchgeführt und ist immer für eine Behandlung gültig. Bei einer Behandlungsdauer > 24 h verliert der Test seine Gültigkeit.

Wird das Gerät nach 24 h nicht ausgeschaltet, weil eine Behandlung läuft und deren Unterbrechung nicht in Frage kommt, etwa weil sie zu einer Gefährdung des Patienten führen kann, so erfolgt nach dieser Zeitspanne auch kein Test der Schutzsysteme. Somit kann der Fall eintreten, dass kein erneuter Test durchgeführt wurde, obwohl die Behandlungsdauer 24 h übersteigt.

Dokumente des Stands der Technik sind US 2016/1151592 A1 und EP 1 838 356 A1. Eine bekannte Dialysemaschine ist beispielsweise die Dialog + Diaysemaschine von B. Braun.

Der vorliegenden Erfindung liegt die Aufgabe zugrunde, ein Blutbehandlungsgerät der eingangs genannten Art dahingehend weiterzubilden, dass die Wahrscheinlichkeit für die Durchführung eines Gerätetest erhöht wird.

Diese Aufgabe wird durch ein Blutbehandlungsgerät mit den Merkmalen des Anspruchs 1 gelöst. Das extrakorporale Blutbehandlungsgerät wird im Folgenden auch einfach als "Gerät" bezeichnet.

Danach ist vorgesehen, dass das Gerät ausgebildet ist, nach einer bestimmten Zeitspanne seit dem Einschalten des Gerätes, d.h. nach einer bestimmten Betriebsdauer mittels der Ausgabeeinheit wenigstens eine Meldung auszugeben, die eine oder mehrere Informationen betreffend die Gültigkeit eines an dem Gerät vorgenommenen Tests betrifft.

Der Nutzer des Gerätes wird somit nach Ablauf einer bestimmten Betriebsdauer des Gerätes über die Gültigkeit eines zuletzt durchgeführten Gerätetests informiert. Der Begriff der "Gültigkeit eines an dem Gerät vorgenommenen Tests" ist weit zu verstehen und umfasst nicht nur die Mitteilung, wie lange oder ob der zuletzt durchgeführte Test noch gültig ist, sondern beispielsweise auch die Aufforderung, innerhalb einer bestimmten Zeitspanne einen Neustart des Gerätes durchzuführen oder das Gerät abzuschalten etc.

Gemäß einer bevorzugten Ausgestaltung der Erfindung ist das Gerät ausgebildet, um nach einer ersten Zeitspanne (z.B. nach 18 h Betriebsdauer) ab dem Einschalten des Gerätes, d.h. nach einer ersten Betriebsdauer, wenigstens eine erste Meldung auszugeben, die die aktuelle Betriebsdauer und/oder die zulässige Betriebsdauer oder die Differenz aus beiden Größen betrifft und/oder die zum Gegenstand hat, dass in einer bestimmten Zeit ein Neustart des Gerätes erforderlich ist, wobei die erste Zeitspanne geringer ist, als die zulässige Betriebsdauer, d.h. als die zulässige Zeitspanne zwischen zwei an dem Blutbehandlungsgerät vorzunehmenden Tests.

Die zulässige Zeitspanne zwischen zwei an dem Gerät vorgenommenen Tests kann beispielsweise 24 h betragen, d.h. nach spätestens 24 h Betriebsdauer ist ein erneuter Gerätetest durchzuführen.

Bei den vorgenannten Werten sowie auch bei den im Folgenden genannten Werten handelt es sich um Beispiele, die die Erfindung nicht beschränken.

Diese Meldung kann in bestimmten Zeitabständen, z.B. stündlich wiederholt werden.

Weiterhin ist erfindungsgemäß vorgesehen, dass das Gerät ausgebildet ist, nach Ablauf einer zweiten Zeitspanne ab dem Einschalten des Gerätes, d.h. nach einer zweiten Betriebsdauer, wenigstens eine zweite Meldung auszugeben, die zum Gegenstand hat, dass das Gerät ausgeschaltet werden muss, wobei die zweite Zeitspanne größer ist als die erste Zeitpanne. Diese zweite Meldung ist somit eine Warnmeldung an den Nutzer, dass das Gerät ausgeschaltet werden muss.

Beträgt die zulässige Betriebsdauer zwischen zwei Tests beispielsweise 24 h, d.h. muss nach spätestens 24 h ein erneuter Test des Gerätes erfolgen, so kann die zweite Zeitspanne beispielsweise 23 h 45 min betragen oder auch größer sein als 24 h. Die zweite Zeitspanne kann somit geringfügig (z.B. 15 min. bis 30 min.) unter der zulässigen Zeitspanne zwischen zwei an dem Gerät vorgenommenen Tests liegen oder diese überschreiten, d.h. in dem vorgenannten Beispiel > 24 h betragen. Auch diese zweite Meldung kann in bestimmten Zeitabständen, z.B. stündlich, wiederholt werden.

Erfindungsgemäß handelt es sich bei dem Gerät um ein Dialysegerät.

Das extrakorporale Blutbehandlungsgerät weist erfindungsgemäß wenigstens eine Sicherheitseinheit auf, die ausgebildet ist, einen erneuten Betrieb des Blutbehandlungsgerätes zu verhindern, wenn kein erneuter Test durchgeführt wurde, d.h. kein gültiger Test vorliegt.

Denkbar ist es weiterhin, dass die zweite Meldung in zwei unterschiedlichen Varianten ausgeführt ist, wobei die erste Variante ausgegeben wird, wenn sich das Gerät in dem Modus der extrakorporalen Blutbehandlung befindet, und wobei die zweite Variante ausgegeben wird, wenn sich das Gerät in einem beliebigen anderen Modus befindet.

Bei der Ausgabeeinheit kann es sich beispielsweise um einen Monitor bzw. Display und/oder um einen Lautsprecher und/oder um einen Sender handeln, mittels dessen die wenigstens eine Meldung an zumindest eine andere Einheit vorzugsweise durch Datenfernübermittlung (z.B. Internet, Mobilfunk etc.) übertragbar ist.

Bei dem genannten Test handelt es sich um den T1-Test für ein Dialysegerät.

Der Test kann derart ausgestaltet sein, dass insbesondere elektrische und/oder elektronische Komponenten bzw. Verbindungen überprüft werden, wir beispielsweise ein ROM-Test, ein RAM-Test, ein Test der Überspannungshardwareabschaltung, ein Test der ADC-Kanäle etc.

Das Gerät kann wenigstens einen Controller aufweisen, der die o.g. Schritte durchführt bzw. veranlasst.

Die vorliegende Offenbarung umfasst des Weiteren ein Verfahren zum Erzeugen einer oder mehrerer Meldungen an einem erfindungsgemäßen extrakorporalen Blutbehandlungsgerät, wobei das Verfahren gemäß den in den Ansprüchen 1 bis 4 genannten Verfahrensschritten abläuft. Das offenbarte Verfahren kann somit einen oder mehrere der in den Ansprüchen 1 bis 4 genannten Maßnahmen umfassen, die durch das erfindungsgemäße extrakorporale Blutbehandlungsgerät ausgeführt werden.

So ist das Verfahren vorzugsweise so ausgeführt, dass nach Ablauf einer bestimmten Zeitspanne seit dem Einschalten des Blutbehandlungsgerätes und/oder bei Eintritt eines bestimmten Ereignisses mittels der Ausgabeeinheit wenigstens eine Meldung ausgegeben wird, die eine oder mehrere Informationen betreffend die Gültigkeit eines an dem Blutbehandlungsgerät vorgenommenen Tests betrifft.

Nach Ablauf einer ersten Zeitspanne kann wenigstens eine erste Meldung ausgegeben werden, die die aktuelle Betriebsdauer und/oder die zulässige Betriebsdauer oder die Differenz aus beiden Größen betrifft oder die zum Gegenstand hat, dass in einer bestimmten Zeit ein Neustart des Blutbehandlungsgerätes erforderlich ist, wobei die erste Zeitspanne geringer ist, als die zulässige Zeitspanne zwischen zwei an dem Blutbehandlungsgerät vorgenommenen Tests.

Weiterhin kann das Verfahren derart ausgeführt sein, dass nach Ablauf einer zweiten Zeitspanne wenigstens eine zweite Meldung ausgegeben wird, die zum Gegenstand hat, dass das Blutbehandlungsgerät ausgeschaltet werden muss, wobei die zweite Zeitspanne größer ist als die erste Zeitpanne. Dabei ist vorzugsweise vorgesehen, dass die zweite Zeitspanne geringfügig unter der zulässigen Zeitspanne zwischen zwei an dem Blutbehandlungsgerät vorgenommenen Tests liegt oder diese überschreitet.

In einer weiteren bevorzugten Ausgestaltung des Verfahrens ist vorgesehen, dass nach Ablauf der ersten Zeitspanne in bestimmten Zeitabständen die erste Meldung erneut ausgegeben wird und/oder nach Ablauf der zweiten Zeitspanne in bestimmten Zeitabständen die zweite Meldung erneut ausgegeben wird.

Des Weiteren kann vorgesehen sein, dass es sich bei dem bestimmten Ereignis um den Übergang des Blutbehandlungsgerätes in den Stand-by-Modus handelt oder um das Ende einer mittels des Blutbehandlungsgerätes vorgenommenen Behandlung handelt. Vorzugsweise erfolgt in diesen Fällen die erste oder die zweite Meldung erst nach Ablauf einer bestimmten Betriebsdauer.

Wie oben ausgeführt, handelt es sich bei dem Blutbehandlungsgerät um ein Dialysegerät, wobei das Verfahren derart ausgebildet ist, dass es sich bei dem bestimmten Ereignis um die Überschreitung der geplanten Behandlungsrestdauer (bzw. die Überschreitung der Ultrafiltrationsrestzeit) über einen Grenzwert handelt, wobei der Grenzwert von der Differenz zwischen der Betriebsdauer des Gerätes bis zur zweiten Meldung und der aktuellen Betriebsdauer abhängt oder durch diese Differenz gebildet wird.

Weiterhin kann das Verfahren so ausgestaltet sein, dass ein erneuter Betrieb des Blutbehandlungsgerätes verhindert wird, wenn kein erneuter Test durchgeführt wurde.

Die Ausgabe der wenigstens einen Meldung an einen Nutzer kann mittels eines Monitors bzw. Displays und/oder eines Lautsprechers und/oder mittels einer Übertragungseinheit erfolgen, mittels derer die wenigstens eine Meldung an zumindest eine andere Einheit übertragbar ist.

Weitere Einzelheiten und Vorteile der Erfindung werden anhand eines im Folgenden beschriebenen Ausführungsbeispiels näher erläutert:
Wird das Gerät vom Anwender oder automatisch (Auto-On Programm, Netzausfall ohne Akku) eingeschaltet, wird die Betriebsdauer, d.h. die Zeit seit dem Einschalten gemessen.

In Zusammenhang mit der Betriebsdauer gibt es die folgenden Meldungen:
Eine erste Meldung mit der Ausgabe der Betriebsdauer und einer Aufforderung zum Ausschalten des Gerätes nach spätestens 24 h Betriebsdauer.

Die erste Meldung könnte lauten:
"Es wurden bereits xy Stunden Betriebsdauer erreicht. Die zulässige Betriebsdauer des Gerätes beträgt 24 h. Vor der nächsten Behandlung das Gerät aus- und wieder einschalten."

Dabei steht "xy" für die aktuelle Betriebsdauer, d.h. die Zeitspanne seit dem Einschalten des Gerätes.

Zusätzlich könnte folgende erste Information an den Nutzer ausgegeben werden:
"Um sicherheitsrelevante Funktionsüberprüfungen automatisch und weitere Behandlungen ohne Unterbrechung durchführen zu können, muss das Gerät nach einer maximalen Betriebsdauer von 24 h aus- und wieder eingeschaltet werden. Die Betriebsdauer ist der Zeitraum vom Einschalten bis zum Ausschalten des Gerätes."

Der Nutzer kann aufgefordert werden, das Lesen dieser Meldung bzw. Information durch das Betätigen einer Taste zu bestätigen.

Die erste Meldung und ggf. die erste Information wird beispielsweise nach 18 h Betriebsdauer an den Nutzer ausgegeben. Dies gilt sowohl während der Behandlung als auch wenn sich das Gerät im Standby-Modus befindet.

Nach der 18 h Betriebsdauer kann die Meldung z.B. stündlich wiederholt werden, bis das Gerät ausgeschaltet wird.

Die erste Meldung und ggf. die erste Information wird auch bei jedem Übergang in den Standby-Modus oder am Ende einer Behandlung ausgegeben, wenn die Betriebsdauer beim Übergang in den Standby-Modus bzw. bei Behandlungsende zumindest 18 h beträgt.

Des Weiteren wird die erste Meldung bereits ab 1 h Betriebsdauer ausgegeben, wenn die UF-Restzeit größer ist als die Differenz zwischen der Betriebsdauer für die zweite Meldung und der aktuellen Betriebsdauer und wenn die verbleibende Zeit bis zur zweiten Meldung noch einen Mindestwert aufweist, z.B. 30 min.

Nach Ablauf der 24 h Betriebsdauer oder kurz vor Ablauf der 24 h Betriebsdauer wird eine zweite Meldung und ggf. eine zweite Information ausgegeben. Anschließend erfolgt eine Wiederholung, z.B. stündlich, bis das Gerät ausgeschaltet wird.

Für die zweite Meldung existieren zwei Varianten, die im Folgenden als 2A und 2B bezeichnet werden.

Meldung 2A wird ausgegeben, wenn die Behandlung läuft und Meldung 2B wird in allen anderen Modi des Gerätes ausgegeben.

Das erste Mal wird die Meldung 2A gemäß diesem Ausführungsbeispiel nach 23 h 45 min ausgegeben, damit der Nutzer ausreichend Zeit hat, eine Behandlung zu beenden.

Die Meldung 2B erscheint auch bei jedem Übergang in den Standby-Modus oder am Ende einer Behandlung, wenn die Betriebsdauer bis zum Übergang in den Standby-Modus bzw. bis zum Behandlungsende zumindest 24 h beträgt.

Im Standby-Modus werden die erste Meldung und die Meldung 2B nur angezeigt, wenn keine Auto-off-Meldung sichtbar ist, aus der der Nutzer entnimmt, dass sich das Gerät nach Ablauf einer bestimmten Zeit im Standby-Modus selbst abschaltet.

Alle Meldungen, d.h. die erste Meldung sowie die Meldungen 2A und 2B sind durch den Nutzer bestätigbar.

Bei jedem Stundensprung erfolgt bei einer Betriebsdauer > 24 h ein Fehlerspeichereintrag, z.B. in Form der Nachricht "Zulässige Betriebsdauer überschritten (xx h)

Die Meldung 2A könnte lauten:
"Zulässige Betriebsdauer des Gerätes überschritten. Es wurden bereits yz Stunden erreicht. Behandlung umgehend beenden und Reinfusion starten! Vor der nächsten Behandlung Gerät aus- und wieder einschalten."

Dabei steht yz für die aktuelle Betriebsdauer des Gerätes, d.h. für die Zeitspanne seit dem letzten Einschalten. Dies gilt entsprechend für die im Folgenden genannte Meldung 2B.

Der ggf. hierzu ausgegebene Info-Text kann dem zu der ersten Meldung genannten Text entsprechen.

Die Meldung 2B könnte lauten:
"Zulässige Betriebsdauer des Gerätes überschritten. Es wurden bereits yz Stunden erreicht. Gerät aus- und wieder einschalten."

Der ggf. hierzu ausgegebene Info-Text kann dem zu der ersten Meldung genannten Text entsprechen.

## Patentansprüche

1. Dialysegerät mit wenigstens einer Ausgabeeinheit zur Ausgabe von zumindest einer Meldung an einen Nutzer des Dialysegeräts, **dadurch gekennzeichnet, dass** das Dialysegerät ausgebildet ist, nach Ablauf einer ersten Zeitspanne seit dem Einschalten des Dialysegeräts mittels der Ausgabeeinheit wenigstens eine erste Meldung auszugeben, die eine oder mehrere Informationen betreffend der Gültigkeit eines an dem Dialysegerät vorgenommenen T1-Tests betrifft, wobei die Information angibt, wie lange der zuletzt durchgeführte T1-Test noch gültig ist,
das Dialysegerät ausgebildet ist, nach Ablauf einer zweiten Zeitspanne wenigstens eine zweite Meldung auszugeben, die zum Gegenstand hat, dass das Dialysegerät ausgeschaltet werden muss, wobei die zweite Zeitspanne größer ist als die erste Zeitspanne, und
das Dialysegerät wenigstens eine Sicherheitseinrichtung aufweist, die ausgebildet ist, einen erneuten Betrieb des Dialysegeräts zu verhindern, wenn kein erneuter T1-Test durchgeführt wurde.

2. Dialysegerät nach Anspruch 1, **dadurch gekennzeichnet, dass** die zweite Zeitspanne geringfügig unter der zulässigen Betriebsdauer, d.h. der zulässigen Zeitspanne zwischen zwei an dem Blutbehandlungsgerät vorgenommenen Tests liegt oder diese überschreitet.

3. Dialysegerät nach einem der Ansprüche 1 bis 2, **dadurch gekennzeichnet, dass** das Dialysegerät ausgebildet ist, nach Ablauf der ersten Zeitspanne in bestimmten Zeitabständen die erste Meldung erneut auszugeben und/oder nach Ablauf der zweiten Zeitspanne in bestimmten Zeitabständen die zweite Meldung erneut auszugeben.

4. Dialysegerät nach einem der vorhergehenden Ansprüche, **dadurch gekennzeichnet, dass** es sich bei der Ausgabeeinheit um ein Display und/oder um einen Lautsprecher und/oder um eine Übertragungseinheit handelt, mittels derer die wengistens eine Meldung an zumindest eine andere Einheit übertragbar ist.

## Claims

1. Dialysis machine comprising at least one output unit for outputting at least one report to a user of the dialysis machine, **characterized in that** the dialysis machine is configured to output at least one first report by means of the output device after the end of a first period of time since the switching on of the dialysis machine, the first report relating to the validity of a T1 test performed on the dialysis machine, the piece of information indicating how long the most recently performed T1 test is valid,
the dialysis machine is configured to output at least one second report after the end of a second period of time, said second report having the content that the dialysis machine has to be switched off, with the second period of time being greater than the first period of time, and
the dialysis machine has at least one safety device that is configured to prevent a repeat operation of the dialysis machine if no T1 test has been carried out anew.

2. Dialysis machine in accordance with claim 1, **characterized in that** the second period of time is slightly below the permitted operating time, i.e. the permitted period of time between two tests carried out at the blood treatment device, or exceeds it.

3. Dialysis machine in accordance with one of the claims 1 to 2, **characterized in that** the dialysis machine is configured to output the first report again at specific time intervals after the end of the first period of time and/or to output the second report again at specific time intervals after the end of the second period of time.

4. Dialysis machine in accordance with one of the preceding claims, **characterized in that** the output unit is a display and/or a loudspeaker and/or a transmission unit by means of which the at least one report can be transmitted to at least one other unit.

## Revendications

1. Appareil de dialyse avec au moins une unité d'émission destinée à émettre au moins un message à un utilisateur de l'appareil de dialyse, **caractérisé en ce que** l'appareil de dialyse est réalisé pour émettre au moyen de l'unité d'émission après l'écoulement d'un premier laps de temps depuis l'activation de l'appareil de dialyse au moins un premier message, qui concerne une ou plusieurs informations concernant la validité d'un test T1 réalisé sur l'appareil de dialyse, dans lequel l'information indique la durée, pendant laquelle le test T1 réalisé en dernier lieu est encore valable,
l'appareil de dialyse est réalisé pour émettre après l'écoulement d'un deuxième laps de temps au moins un deuxième message, qui a pour objet que l'appareil de dialyse doit être impérativement désactivé, dans lequel le deuxième laps de temps est supérieur au premier laps de temps, et
l'appareil de dialyse présente au moins un système de sécurité, qui est réalisé pour empêcher une remise en marche de l'appareil de dialyse lorsqu'aucun nouveau test T1 n'a été effectué.

2. Appareil de dialyse selon la revendication 1, **caractérisé en ce que** le deuxième laps de temps est légèrement inférieur à la durée de fonctionnement autorisée, en d'autres termes au laps de temps autorisé entre deux tests réalisés sur l'appareil de traitement du sang, ou dépasse celle-ci.

3. Appareil de dialyse selon l'une quelconque des revendications 1 à 2, **caractérisé en ce que** l'appareil de dialyse est réalisé pour émettre à nouveau le premier message à des intervalles de temps définis après l'écoulement du premier laps de temps et/ou pour émettre à nouveau le deuxième message à des intervalles de temps définis après l'écoulement du deuxième laps de temps.

4. Appareil de dialyse selon l'une quelconque des revendications précédentes, **caractérisé en ce que** l'unité d'émission est un système d'affichage et/ou un haut-parleur et/ou une unité de transmission, au moyen duquel/de laquelle l'au moins un message peut être transmis à au moins une autre unité.
